# EUROPÄISCHE PATENTSCHRIFT

(11) **EP 2 895 431 B1**
(45) Veröffentlichungstag und Bekanntmachung des Hinweises auf die Patenterteilung: **11.10.2017**
(21) Anmeldenummer: 13765668.2
(22) Anmeldetag: 12.09.2013
(51) Int. Cl.: C02F 3/06, C02F 3/10, C11B 1/10, C12N 1/02, C12N 1/06, C02F 1/26, C02F 3/04, C02F 3/28, C02F 3/32

(54) **VERFAHREN ZUR FEST-FLÜSSIGTRENNUNG EINER SUSPENSION ENTHALTEND MIKROALGEN**
METHOD FOR THE SOLID/LIQUID SEPARATION OF A SUSPENSION CONTAINING MICROALGAE
PROCÉDÉ DE SÉPARATION SOLIDE/LIQUIDE D'UNE SUSPENSION CONTENANT DES MICROALGUES

(30) Priorität: 13.09.2012 DE 102012216339
(43) Veröffentlichungstag der Anmeldung: 22.07.2015
(73) Patentinhaber: Gicon Grossmann Ingenieur Consult GmbH, 01219 Dresden (DE)
(72) Erfinder: GROSSMANN, Jochen, 01187 Dresden (DE); COTTA, Fritz, 06217 Merseburg (DE); MATSCHKE, Martin, 07973 Greitz (DE); GRIEHL, Carola, 06118 Halle / S. (DE)
(74) Vertreter: Kailuweit & Uhlemann Patentanwälte Partnerschaft mbB
(86) Internationale Anmeldenummer: PCT/EP2013/068895
(87) Internationale Veröffentlichungsnummer: WO 2014/041063

(56) Entgegenhaltungen:
- WO-A2-2012/034118
- US-A1- 2011 036 778
- US-A1- 2012 024 769

## Beschreibung

Die Erfindung betrifft ein Verfahren zur Fest-Flüssigtrennung von Suspensionen enthaltend Mikroalgen wobei als Filtermaterial biologisch abbaubare Materialien verwendet werden.

Nach dem aktuellen Stand der Technik können Mikroalgen stofflich oder energetisch bzw. durch Kopplung verschiedener Technologien stofflich und energetisch verwertet werden. In jedem Fall ist im ersten Verfahrensschritt eine Fest-Flüssig-Trennung erforderlich nach der, in Abhängigkeit der Trennleistung, eine konzentrierte Suspension oder Mikroalgenpaste erhalten wird.

Ausgenommen der Sprüh-/Gefriertrocknung, erfolgt die Fest-Flüssig-Trennung durch Filtration mittels Filterpapiere oder Filtervliese, durch Separation (z.B. Tellerseparator), Zentrifugation oder Dekantation. Optional kann eine Flockung der Mikroalgen vorgeschaltet werden, die die nachfolgende Trennung erleichtert. Allen genannten Verfahren gemein sind hohe Betriebskosten, verursacht durch die aufzuwendende Energie für die verfahrenstechnisch geforderten Temperatur- und/oder Druckunterschiede. Weiterhin werden bei den Filtrierverfahren teure Hilfsmittel benötigt, die nach dem Verfahren gegebenenfalls entsorgt werden müssen.

Die DE 10 2005 026 372 A1 beschreibt eine mechanische Aufbereitungsstufe zur Aufbereitung von Gärresten aus der Biogaserzeugung, welche eine Wendel-Filterpresse und einen Zyklonfilter vorsieht. Dadurch ist eine effektive Fest-Flüssigtrennung bis zu einer Partikelgröße von 10 µm möglich. Jedoch ist bei dieser Art der Fest-Flüssigtrennung ein erheblicher Energieaufwand notwendig.

EP 2,322,262 A1 beschreibt einen Filter in Wasserbehandlungs- und Aufbereitungsanlagen zur Abtrennung von organischen Schwebstoffen und Algen. Das Filtermaterial besteht dabei aus einer Körnung von ladungstechnisch neutralem Glas und ist auf die Korngrößenverteilung der zu filtrierenden Schmutzstoffe abgestimmt.

DE 102009039554 A1 beschreibt den Einsatz eines Membranfilters zur Trennung bzw. Vorkonzentrierung von Mikroalgensuspensionen. Die Filtrate können anschließend wieder der Kultivierung zugeführt werden. Der Restwassergehalt der konzentrierten Mikroalgensuspension beträgt, je nach Einsatz eines Flockungsmittels und vorliegender Algenspezies, zwischen 60% und 95%.

Im Fall einer Wertstoffgewinnung sind die Zellmembranen der Mikroalgen zu zerstören, um die Inhaltsstoffe zugänglich zu machen. Nach dem Stand der Technik wird der Mikroalgen-Aufschluss enzymatisch, mechanisch, durch Extrusion, mittels Ultraschall oder Mikrowellen durchgeführt. Wie bei der Fest-Flüssig-Trennung, werden auch für diese Techniken enorme Energiemengen oder Hilfsmittel benötigt, die das Gesamtverfahren in seiner Kostenbilanz sehr negativ beeinflussen.

WO2012/034118 beschreibt ein Verfahren zur Trennung von Suspensionen enthaltend Mikroorganismen mit einem selbstverbrauchenden Filter umfassend eine Filterschicht aus biologisch abbaubarem Material. Die Aufgabe der Erfindung besteht daher darin, ein Verfahren zur Abtrennung von Mikroalgen aus der Kultivierungssuspension anzugeben, welches eine Minimierung der Kosten bei der Isolierung von Inhaltsstoffen aus Mikroalgen ermöglicht.

Die Aufgabe wird durch ein Verfahren gemäß dem Hauptanspruch 1 gelöst. Vorteilhafte Ausgestaltungen sind in den abhängigen Ansprüchen angegeben.

Erfindungsgemäß wird ein Verfahren zur Fest-Flüssigtrennung einer Suspension, umfassend die folgenden Schritte vorgeschlagen: Zunächst wird eine Filterschicht aus organischem Material in eine Filtervorrichtung eingebracht. Danach erfolgt zumindest eine Zugabe der zu trennenden Suspension auf die Filterschicht, wobei die festen Bestandteile der Suspension auf der Filterschicht abgelagert werden und der flüssige Bestandteil als Filtrat die Filterschicht passiert. Die festen Bestandteile der Suspension umfassen Mikroalgen, wobei nachfolgend eine Hydrolyse des Filtermaterials und der Mikroalgen durch im Filtermaterial natürlich enthaltene oder separat zugesetzte Mikroorganismen erfolgt. Für die Hydrolyse ist es dabei wesentlich, dass die Filterschicht feucht gehalten wird, um ein Austrocknen der Mikroalgen zu vermeiden. Hierzu kann beispielsweise Flüssigkeit, Filtrat oder Suspension zugegeben werden. Der Feuchtegehalt der Filterschicht sollte zwischen 30-90% liegen. Die organische Trockensubstanz (OTS) der Filterschicht beträgt 10-70 Gew.%, bevorzugt 20-60 Gew.%. Die organische Trockensubstanz (OTS) ist der Anteil der organischen Bestandteile eines Stoffes, nach vollständigem Entzug von Wasser und aller mineralischer Bestandteile.

Die Hydrolyse, sowohl der Filterschicht, als auch der darin oder darauf abgelagerten Mikroalgen erfolgt durch Mikroorganismen, die separat zugegeben werden müssen oder natürlichen Ursprungs sind und dadurch mit dem organischen Substrat der Filterschicht in das System eingebracht werden. Unter natürlichem Ursprung wird dabei verstanden, dass die Mikroorganismen auf dem organischen Substrat bereits vorhanden sind.

In einer Ausführungsform der Erfindung erfolgt eine wiederholte Zugabe der Suspension auf die Filterschicht. Da die Mikroalgen in der Suspension je nach verwendeter Art 2-10 mal schneller hydrolysieren als die Filterschicht, können so mehrere Suspensionszugaben bis zur vollständigen Umsetzung des Filtermaterials erfolgen.

In einer weiteren Ausführungsform der Erfindung umfasst das organische Material biologisch abbaubares Material, Grünschnitt, Hackschnitzel aus Holz oder andere Pflanzen oder Silagen. Der eigentliche Filter besteht aus organischem, zumindest teilweise abbaubarem Material, wie z.B. Grünschnitt, Hackschnitzel aus Holz, Mais, Silagen o.ä. Das Schüttgewicht des eingesetzten Materials sollte 0,8 - 1,2 t/m³ betragen, um die gewünschten Filtereigenschaften zu gewährleisten.

In einer weiteren Ausführungsform der Erfindung werden als Mikroalgen z.B. *Scenedesmus rubescens, Chlorella vulgaris* oder *Chlorella sorokiniana* verwendet.

In einer weiteren Ausführungsform der Erfindung erfolgt die Hydrolyse in der Filtervorrichtung unter aeroben und/oder anaeroben Bedingungen. Dabei ist beispielsweise ein Wechsel zwischen anaerober und aerober Verfahrensweise denkbar.

In einer weiteren Ausführungsform der Erfindung weist die Filtervorrichtung eine zylinder-, kugel- oder quaderförmige Bauform mit Zulauf, Ablauf und Austragsöffnung auf, wobei optional ein Feststoff-Rückhaltenetz vorgesehen werden kann. Die Filtereinheit besteht aus einem zylindrischen, kugel- oder quaderförmigen Gefäß, welches an der Unterseite verschlossen und mit einem Abfluss versehen ist. Weiterhin ist eine Haltevorrichtung, z.B. Drahtgeflecht (Zwischenboden) im unteren Teil des Gefäßes angebracht, welche die Schüttung, also den eigentlichen Filter trägt. Optional kann das Gefäß mit einem Verschluss für den anaeroben Betrieb verschlossen und mit einer Reinigungsklappe an der Mantelwandung ausgestattet werden.

In einer weiteren Ausführungsform der Erfindung werden die durch die Hydrolyse gebildeten löslichen, organischen und anorganischen Bestandteile aus der Filterschicht herausgewaschen.

In einer weiteren Ausführungsform der Erfindung erfolgt der Waschvorgang mit rückgeführtem Filtrat oder durch erneute Zugabe von Suspension. Dadurch werden zum einen die durch die Hydrolyse gebildeten löslichen, organischen und anorganischen Bestandteile aus der Filterschicht herausgewaschen, zum anderen eine Befeuchtung des Filtermaterials bewirkt. Bei der Zugabe von Suspension werden zudem neue Mikroorganismen in die Filterschicht eingetragen.

In einer weiteren Ausführungsform der Erfindung werden allein durch den Hydrolysevorgang die Inhaltsstoffe der Mikroalgen freigesetzt. Die Hydrolyse erfolgt dabei durch die Mikroorganismen, welche, separat zugegeben werden müssen oder natürlichen Ursprungs sind und dadurch mit dem organischen Substrat der Filterschicht in das System eingebracht werden. Durch die Hydrolyse der Mikroalgen durch die Mikroorganismen wird eine Zugabe von kommerziell erhältlichen Enzymen vermieden. Zudem werden technische Anlagen für einen herkömmlichen Aufschluss der Mikroalgen mittels Extrusion, Ultraschall, Mikrowellen oder dergleichen vermeiden. Die durch die Hydrolyse der Mikroalgen freigesetzten Wertstoffe werden über die Zugabe von rückgeführtem Filtrat oder Zugabe von Suspension in das Filtrat eingetragen und stehen somit einer nachfolgenden Extraktion zur Verfügung. Erfindungsgemäß erfolgt eine Extraktion von Wertstoffen aus dem Filtrat. Dabei kann es sich etwa um lösliche organische Verbindungen wie etwa Carotinoide, Lipide oder Proteine handeln. Denkbar ist auch eine Ölabscheidung.

In einer weiteren Ausführungsform der Erfindung wird das Filtrat zur Erzeugung von Biogas in einem Methanreaktor verwendet. Dabei wird in einem Methanisierungsreaktor aus den noch enthaltenen, organischen Reststoffen Biogas erzeugt. Die Ablauge aus dem Methanreaktor, welche noch anorganische Salze enthält, kann nach der Dekontamination wieder zur Mikroalgenkultivierung eingesetzt werden. Damit ist der Aufbau eines integrierten Stoffkreislaufsystems möglich, welches vor allem durch den Einsatz eines selbstverbrauchenden Biofilters besonders ökonomisch und effizient arbeitet.

In einer weiteren Ausführungsform der Erfindung erfolgt eine kontinuierliche Öl-/Lipidabtrennung aus dem Filtrat, sodass lediglich ein Verfahrensschritt zwischen Suspension und Rohöl benötigt wird.

In einer weiteren Ausführungsform der Erfindung wird das Filtrat sowohl stofflich (z.B. Extraktion von Wertstoffen), als auch energetisch (z.B. Erzeugung von Biogas in einem sich anschließenden Methanreaktor) verwertet.

In einer weiteren Ausführungsform der Erfindung wird das verbleibende, nicht oder nur schwer biologisch abbaubare Filtermaterial als organischer Kompost verwendet. Die nicht hydrolysierten Bestandteile der Filterschicht werden der Kompostierung zugeführt und dort verwertet.

Ein weiterer Gegenstand der Erfindung ist auch die Verwendung einer Filtervorrichtung zur Abtrennung von Mikroalgen aus ihrer Kultivierungssuspension, Hydrolyse der Mikroalgen und zur Isolierung von Inhaltsstoffen aus Mikroalgen, wobei die Filtervorrichtung ein selbstverbrauchender Filter mit einer Filterschicht aus biologisch abbaubarem Material ist, mit einem Schüttgewicht von 0.8-1.2 t/m³. Erfindungsgemäß wird die Aufgabenstellung durch Einsatz eines selbstverbrauchenden Biofilters gelöst. Nach erfolgter Fest-Flüssig-Trennung wird sowohl der Filter, als auch der Filterkuchen unter stetiger, dosierter Hydrolysatrückführung mit zunehmender Dauer hydrolysiert. Im Ergebnis wird ein Stoffgemisch aus organischen Säuren, stammend aus dem organischen Filtermaterial/Mikroalgen, und anorganischen Salzen erhalten. Zusätzlich enthält das Stoffgemisch Wertstoffe aus den Mikroalgen (Farbstoffe, Lipide, Proteine), welche in nachfolgenden Prozessschritten direkt isoliert werden können.

Der Nutzen der Erfindung besteht darin, dass das eingesetzte organische Material kostengünstig isoliert und, sowohl stofflich, als auch energetisch weiterverarbeitet werden kann. Während nicht hydrolysierbares Material als Kompost verwendet wird, wird das Hydrolysat entweder direkt oder nach einer Extraktion bzw. Ölabscheidung, vorzugsweise in einen Methanreaktor gegeben und Biogas erzeugt. Diese Verfahrensweise führt bei der Abtrennung der Mikroalgen, deren Aufschluss, sowie der Isolation der Inhaltsstoffe zu einer Kostenreduktion von über 50%. Aufgrund dessen könnte die Mikroalgentechnologie im industriellen Maßstab durch Anwendung dieses Verfahrens auch für die Produktion tief- und mittelpreisiger Produkte, sowie für eine wirtschaftliche, energetische Verwertung der Mikroalgenbiomasse interessant sein.

Zur Lösung der Aufgabe ist es auch denkbar, die vorbeschriebenen Ausführungsformen zweckmäßig miteinander zu kombinieren.

Nachfolgend soll die Erfindung anhand einiger Ausführungsbeispiele und zugehöriger Figuren eingehender erläutert werden. Die Ausführungsbeispiele sollen dabei die Erfindung beschreiben ohne diese zu beschränken.

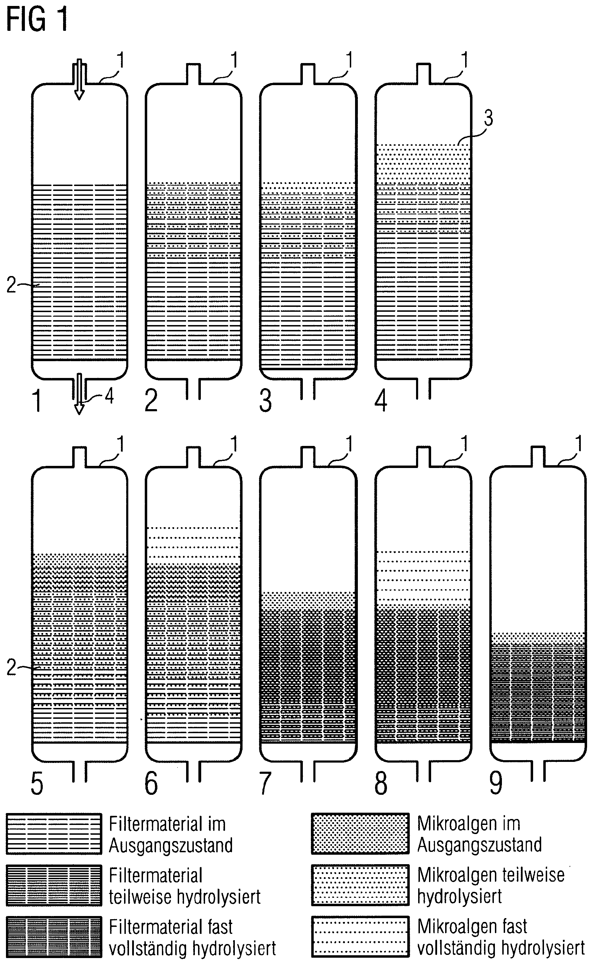

Es zeigt die Fig. 1 (1-9) eine schematische Darstellung des erfindungsgemäßen Verfahrens.

In einem ersten Ausführungsbeispiel ist in Fig. 1 schematisch die erste Phase des erfindungsgemäßen Verfahrens dargestellt. Dabei wird in Fig. 1 (1) die Filtervorrichtung **1,** welche beispielsweise zylinderförmig ausgeführt ist, eine Filterschicht **2** eingebracht. Die Filterschicht **2** besteht aus organischem, zumindest teilweise abbaubarem Material, wie z.B. Grünschnitt, Hackschnitzel aus Holz, Mais o.ä. oder Silagen. Das Schüttgewicht des eingesetzten Materials sollte 0,8 - 1,2 t/m³ betragen, um die gewünschten Filtereigenschaften zu gewährleisten.

In Fig. 1 (2) wird auf die Filterschicht **2** direkt die (ggf. geflockte) Mikroalgensuspension **3** mit 50 - 300 I/min und m² Grundfläche, je nach Korngröße des eingesetzten Filterschichtmaterials gegeben. Dabei werden die Mikroalgen **3** durch das Filtermaterial in der Filterschicht **2** zurückgehalten und damit die Porosität der Filterschicht **2** verringert. Das Filtrat **4** tritt an der Unterseite der Filtervorrichtung **1** aus und wird aufgefangen.

Durch Zugabe weiterer Mikroalgensuspension **3** bildet sich ein Filterkuchen aus Mikroalgen **3** über der Filterschicht **2** (Fig. 1 (3)). Nach dem Aufbau einer Filterkuchenschicht über dem Filtermaterial, kann entsprechend des Gefäßvolumens die Mikroalgensuspension schneller zugegeben werden. Beim Erreichen des maximalen Füllstandes wird die Zugabe gestoppt (Fig. 1 (4)). Nach der Beladung des Filters beginnt die Hydrolyse des organischen Materials. Durch stetige Rückführung/Abtrennung des Filtrats werden dabei die entstandenen Verbindungen, wie organische Säuren oder freigesetzte Inhaltsstoffe der Mikroalge aus dem Filterkuchen/Filtermaterial gewaschen.

In Fig. 1 (5-9) ist der weitere Verlauf des Verfahrens dargestellt. Je nach Mikroalgenspezies werden diese innerhalb von 2 - 10 Tagen unter stetiger Zugabe von Wasser bzw. Rückführung von Filtrat **4** hydrolysiert, die Zellmembranen abgebaut und Wertstoffe der Mikroalge freigesetzt (Fig. 1 (5). Der Abbau des Filtermaterials **2** unter Freisetzung z.B. organischer Säuren beginnt ebenfalls. Die Hydrolyse der eingesetzten Mikroalgen **3** erfolgt je nach Spezies etwa 2 - 10 mal schneller, als diese des Filtermaterials **2,** sodass entsprechend Mikroalgensuspension **3** nachdosiert werden kann.

Da die Mikroalgen **3** im Vergleich zum Filtermaterial **2** deutlich schneller hydrolysiert werden, wird erneut Mikroalgensuspension **3** auf den Filter **2** gegeben. Je nach Wertstoffgehalt des bisher gewonnenen Filtrates **4,** sowie der Stabilität der darin enthaltenen Verbindungen wird Filtrat **4** weiterhin rückgeführt, bzw. durch Filtrat **4** mit niedrigem Wertstoffgehalt ersetzt. (Fig. 1 (6,7)). Das Filtermaterial **2** wurde bereits merklich abgebaut (Fig. 1 (8).

Der Vorgang der Zugabe von Mikroalgensuspension **3** wird solange wiederholt, bis der Abbau des Filtermaterials **2** zum Stillstand kommt. Nach erfolgter vollständiger Hydrolyse des eingesetzten organischen Materials und Auswaschen der Wertstoffe wird die Filtervorrichtung **1** entleert, gereinigt und der Inhalt als Kompost verwendet. Der Filterapparat **1** wird nun erneut befüllt, sodass ein Zyklus etwa 20 - 40 Tage, je nach Abbaubarkeit des eingesetzten Materials dauert (Fig. 1 (9)).

Das erhaltene Filtrat **4** wird je nach Wertstoffgehalt rückgeführt bzw. weiterverarbeitet. Im Fall einer Öl-/Lipidgewinnung ist eine kontinuierliche Wertstoffabtrennung und Rückführung der wässerigen Phase, je nach Anlagengröße, möglich. Andernfalls können enthaltene Wertstoffe, z.B. Carotinoide, Proteine oder auch Lipide mittels Extraktion o.ä. isoliert werden.

Nachdem das Filtrat **4** stoffwirtschaftlich aufgearbeitet und die gewünschten Inhaltsstoffe isoliert wurden, erfolgt nun vorzugsweise die energetische Nutzung der wässerigen Ablauge. In einem nicht näher dargestellten Methanisierungsreaktor wird aus den in der wässrigen Ablauge noch enthaltenen, organischen Reststoffen Biogas erzeugt. Die Ablauge aus dem Methanreaktor, welche noch anorganische Salze enthält, kann nach der Dekontamination wieder zur Mikroalgenkultivierung eingesetzt werden. Damit ist der Aufbau eines integrierten Stoffkreislaufsystems möglich, welches vor allem durch den Einsatz eines selbstverbrauchenden Biofilters besonders ökonomisch und effizient arbeitet.

### Bezugszeichenliste

- 1: Filtervorrichtung 2 Filterschicht
- 3: Mikroalgen 4 Filtrat

## Patentansprüche

1. Verfahren zur Abtrennung von Mikroalgen aus ihrer Kultivierungssuspension, umfassend die Schritte:
- Einbringen einer Filterschicht (2) aus organischem Material in eine Filtervorrichtung (1), wobei das organische Material biologisch abbaubares Material, Grünschnitt, Hackschnitzel aus Holz oder anderen Pflanzen oder Silagen umfasst, wobei der Feuchtegehalt der Filterschicht (2) zwischen 30-90% liegt,
- zumindest eine Zugabe einer zu trennenden Kultivierungssuspension (3) von Mikroalgen auf die Filterschicht (2), wobei die festen Bestandteile der Suspension (3) auf der Filterschicht (2) abgelagert werden und der flüssige Bestandteil als Filtrat (4) die Filterschicht passiert, wobei die festen Bestandteile der Suspension (3) Mikroalgen umfassen, wobei eine Hydrolyse des Filtermaterials und der Mikroalgen durch im Filtermaterial natürlich enthaltene oder separat zugesetzte Mikroorganismen erfolgt,
- Extraktion von Wertstoffen aus Mikroalgen aus dem Filtrat (4).

2. Verfahren nach Anspruch 1 weiterhin umfassend, dass eine wiederholte Zugabe der Suspension (3) auf die Filterschicht (2) erfolgt.

3. Verfahren nach einem der vorhergehenden Ansprüche, **dadurch gekennzeichnet, dass** die Hydrolyse in der Filtervorrichtung (1) unter aeroben und/oder anaeroben Bedingungen erfolgt.

4. Verfahren nach einem der vorhergehenden Ansprüche, **dadurch gekennzeichnet, dass** die Filtervorrichtung (1) eine zylinder-, kugel- oder quaderförmige Bauform mit Zulauf, Ablauf und Austragsöffnung aufweist.

5. Verfahren nach einem der vorhergehenden Ansprüche, **dadurch gekennzeichnet, dass** die durch die Hydrolyse gebildeten löslichen, organischen und anorganischen Bestandteile aus der Filterschicht (2) herausgewaschen werden.

6. Verfahren nach einem der vorhergehenden Ansprüche, **dadurch gekennzeichnet, dass** der Waschvorgang mit rückgeführtem Filtrat (4) oder durch erneute Zugabe von Suspension (3) erfolgt.

7. Verfahren nach einem der vorhergehenden Ansprüche, **dadurch gekennzeichnet, dass** allein durch den Hydrolysevorgang Inhaltsstoffe der Mikroalgen (3) freigesetzt werden.

8. Verfahren nach einem der vorhergehenden Ansprüche, **dadurch gekennzeichnet, dass** das Filtrat (4) zur Erzeugung von Biogas in einem Methanreaktor verwendet wird.

9. Verfahren nach einem der vorhergehenden Ansprüche, **dadurch gekennzeichnet, dass** eine kontinuierliche ÖI-/Lipidabtrennung aus dem Filtrat (4) erfolgt, sodass lediglich ein Verfahrensschritt zwischen Suspension und Rohöl benötigt wird.

10. Verfahren nach einem der vorhergehenden Ansprüche, **dadurch gekennzeichnet, dass** nicht oder nur schwer biologisch abbaubares Filtermaterial (2) als organischer Kompost verwendet wird.

11. Verwendung einer Filtervorrichtung (1) in einem Verfahren nach einem der Ansprüche 1 bis 10 zur Abtrennung von Mikroalgen aus ihrer Kultivierungssuspension, Hydrolyse der Mikroalgen und zur Isolierung von Inhaltsstoffen aus Mikroalgen, wobei die Filtervorrichtung (1) ein selbstverbrauchender Filter mit einer Filterschicht (2) aus biologisch abbaubarem Material ist, mit einem Schüttgewicht von 0.8-1.2 t/m³.

## Claims

1. A method for separating microalgae from their cultivation suspension comprising the steps:
- introduction of a filter layer (2) made of organic material into a filter device (1), wherein the organic material comprises biodegradable material, green waste, wood chips or other plants or silage, wherein the moisture content of the filter layer (2) lies between 30-90%,
- at least one addition of a cultivation suspension (3) of microalgae to be separated onto the filter layer (2), wherein the solid components of the suspension (3) are deposited on the filter layer (2) and the liquid component passes through the filter layer as the filtrate (4), wherein
the solid constituents of the suspension (3) comprise microalgae, wherein hydrolysis of the filter material and the microalgae by microorganisms naturally contained in the filter material or separately added takes place,
- extraction of resources made of microalgae from the filtrate (4).

2. The method according to claim 1, furthermore comprising a repeated addition of the suspension (3) to the filter layer (2).

3. The method according to one of the preceding claims, **characterized in that** the hydrolysis in the filter device (1) takes place under aerobic and/or anaerobic conditions.

4. The method according to one of the preceding claims, **characterized in that** the filter device (1) has a cylindrical, spherical or rectangular design with an inlet, outlet and discharge opening.

5. The method according to one of the preceding claims, **characterized in that** the soluble organic and inorganic components are washed out of the filter layer (2) by hydrolysis.

6. The method according to one of the preceding claims, **characterized in that** the washing process takes place with recycled filtrate (4) or through the renewed addition of suspension (3).

7. The method according to one of the preceding claims, **characterized in that** constituents of the microalgae (3) are released simply by the hydrolysis process.

8. The method according to one of the preceding claims, **characterized in that** the filtrate (4) is used for the production of biogas in a methane reactor.

9. The method according to one of the preceding claims, **characterized in that** a continuous oil/lipid separation from the filtrate (4) takes place, so that only one method step between the suspension and crude oil is required.

10. The method according to one of the preceding claims, **characterized in that** filter material (2) that is non-biodegradable or is not readily biodegradable is used as the organic compost.

11. Use of a filter device (1) in a method according to one of claims 1 to 10 for the separation of microalgae from their cultivation suspension, hydrolysis of the microalgae and for the isolation of constituents of microalgae, wherein the filter device (1) is a self-consuming filter with a filter layer (2) made of biodegradable material with a bulk density of 0.8 - 1.2 t/m³.

## Revendications

1. Procédé destiné à séparer des microalgues à partir d'une suspension de culture, comprenant les étampes :
- mise en place d'une couche de filtrage (2) en matériau organique dans un dispositif de filtrage (1), le matériau organique comprenant un matériau biodégradable, des déchets verts, des copeaux de bois ou d'autres végétaux ou d'ensilages, la teneur en humidité de la couche de filtrage (2) étant de 30-90%,
- au moins une addition d'une suspension de culture (3) de microalgues à séparer sur la couche de filtrage (2), les composants solides de la suspension (3) étant déposés sur la couche de filtrage (2) et le composant liquide traverse la couche de filtrage en tant que filtrat (4), les composants solides de la suspension (3) comprenant des microalgues, une hydrolyse du matériau de filtre et des microalgues ayant lieu par les microorganismes contenus naturellement dans le matériau de filtre ou séparément ajoutés,
- extraction des matières des microalgues du filtrat (4).

2. Procédé selon la revendication 1 comprenant en plus qu'une addition répétée de la suspension (3) a lieu sur la couche de filtrage (2).

3. Procédé selon l'une quelconque des revendications précédentes, **caractérisé en ce que** l'hydrolyse dans le dispositif de filtrage (1) a lieu dans des conditions aérobies et/ou anaérobies.

4. Procédé selon l'une quelconque des revendications précédentes, **caractérisé en ce que** le dispositif de filtrage (1) comporte une forme de construction cylindrique, sphérique ou parallélépipédique avec, admission, évacuation et ouverture d'extraction.

5. Procédé selon l'une quelconque des revendications précédentes, **caractérisé en ce que** les composants solubles formés par l'hydrolyse, organiques et minéraux sont extraits par lavage de la couche de filtrage (2).

6. Procédé selon l'une quelconque des revendications précédentes, **caractérisé en ce que** l'opération de lavage a lieu avec filtrat recyclé (4) ou par addition renouvelée de suspension (3).

7. Procédé selon l'une quelconque des revendications précédentes, **caractérisé en ce que** les constituants des microalgues (3) ne sont libérés que par l'opération d'hydrolyse.

8. Procédé selon l'une quelconque des revendications précédentes, **caractérisé en ce que** le filtrat (4) est utilisé pour produire du biogaz dans un réacteur de méthane.

9. Procédé selon l'une quelconque des revendications précédentes, **caractérisé en ce qu'**une séparation continue huile/lipide du filtrat (4) a lieu de telle manière qu'une seule étape de procédé est nécessaire entre la suspension et le pétrole brut.

10. Procédé selon l'une quelconque des revendications précédentes, **caractérisé en ce que** du matériau de filtrage (2) non ou uniquement difficilement biodégradable est utilisé comme composte organique.

11. Utilisation d'un dispositif de filtrage (1) dans un procédé selon l'une quelconque des revendications 1 à 10 pour la séparation des microalgues de leur suspension de culture, l'hydrolyse des microalgues et pour l'isolation des constituants des microalgues, le dispositif de filtrage (1) étant un filtre autoconsommant avec une couche de filtrage (2) en matériau biodégradable, avec une densité apparente de 0,8 - 1,2 t/m³.
